(19) Europäisches Patentamt
European Patent Office
Office européen des brevets

(11) Veröffentlichungsnummer : **0 107 138**
**B1**

(12) # EUROPÄISCHE PATENTSCHRIFT

(45) Veröffentlichungstag der Patentschrift : **09.09.87**

(51) Int. Cl.⁴ : **C 07 D 501/46**, A 61 K 31/545

(21) Anmeldenummer : **83110116.7**

(22) Anmeldetag : **11.10.83**

(54) **Neue Cephalosporine und Verfahren zu ihrer Herstellung.**

(30) Priorität : **23.10.82 DE 3239365**

(43) Veröffentlichungstag der Anmeldung : **02.05.84 Patentblatt 84/18**

(45) Bekanntmachung des Hinweises auf die Patenterteilung : **09.09.87 Patentblatt 87/37**

(84) Benannte Vertragsstaaten : **AT BE CH DE FR GB IT LI NL SE**

(56) Entgegenhaltungen :
**EP-A- 0 049 448**
**EP-A- 0 081 674**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Kinast, Günther, Dr.**
**Am Eckbusch 35**
**D-5600 Wuppertal 1 (DE)**
Erfinder : **Boberg, Michael, Dr.**
**Untere Bergerheide 47**
**D-5600 Wuppertal 1 (DE)**
Erfinder : **Metzger, Karl Georg, Dr.**
**Pahlkestrasse 75**
**D-5600 Wuppertal 1 (DE)**
Erfinder : **Zeiler, Hans-Joachim, Dr.**
**Elsbeeker Strasse 46**
**D-5620 Velbert 15 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Cephalosporine, ihre Verwendung als Arzneimittel, insbesondere in der antibakteriellen Therapie sowie Verfahren zu ihrer Herstellung.

Es sind aus der europäischen Patentanmeldung 0 049 448-A2 β-Lactamantibiotika der Formel

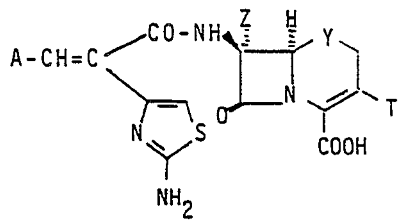

worin T Pyridiniummethyl, 4-Carbamoylpyridiniummethyl u. ä. bedeutet, bekannt.

Durch die Erfindung werden Cephalosporine der allgemeinen Formel I zur Verfügung gestellt.

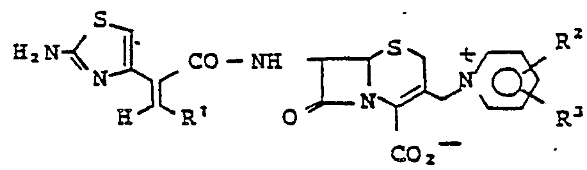

(I)

In der allgemeinen Formel I bedeuten

$R^1$ = $C_1$-$C_6$ Alkyl, Phenyl oder Halogen-substituiertes Phenyl,

$R^2$ und $R^3$ = verschieden oder gleich = H, $C_1$-$C_4$-Alkyl, Chlor, Brom, Carbamoyl, N—$C_1$-$C_4$-Alkylcarbamoyl.

Bevorzugte Verbindungen sind solche mit

$R^1$ = $C_1$-$C_5$ Alkyl

$R^2$ = H, $C_1$-$C_2$-Alkyl, Carbamoyl, Chlor, Brom.

Ganz besonders bevorzugt ist die Verbindung mit

$R^1$ = $CH_3$

$R^2$, $R^3$ = H.

Die Verbindungen der Formel I erhält man, indem man die Säuren der Formel II, worin $R^4$ eine übliche Schutzgruppe darstellt, in die gemischten Anhydride der Formel III überführt und diese mit den bekannten Verbindungen der Formel IV umsetzt und anschließend von den erhaltenen Verbindungen der Formel V die Schutzgruppe $R^4$ abspaltet.

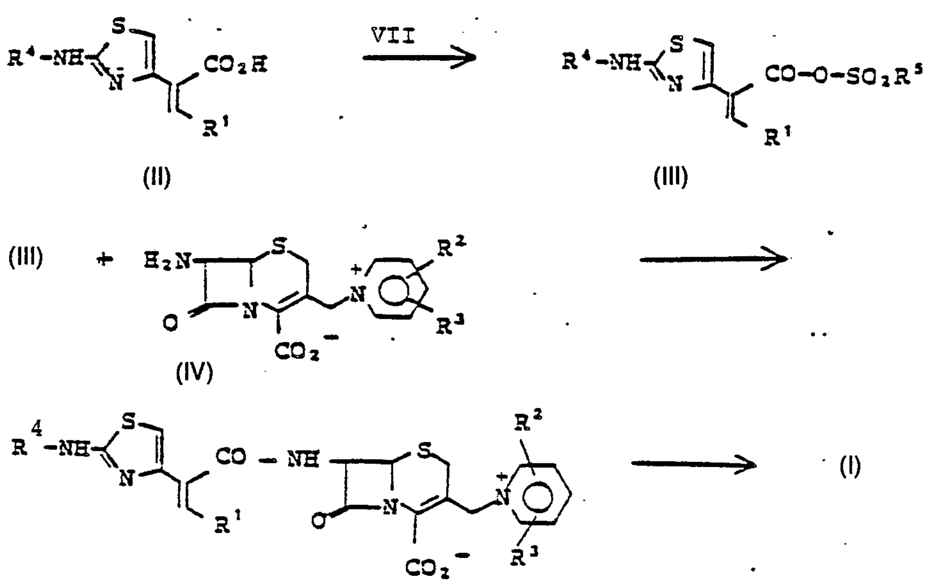


(II) (III)

(III) +

(IV)

(I)

Für das Verfahren ist es vorteilhaft, mit $R^4$ eine säurelabile Schutzgruppe wie z. B. tert.-Butyloxycarbonyl, Trityl oder Formyl zu verwenden und die Abspaltung von $R^4$ in V mit z. B. Trifluoressigsäure oder Ameisensäure vorzunehmen.

Weiterhin ist es vorteilhaft, $R^5=CH_3$ zu wählen.
Die Verbindungen II und III lassen sich nach folgendem Formelschema herstellen.

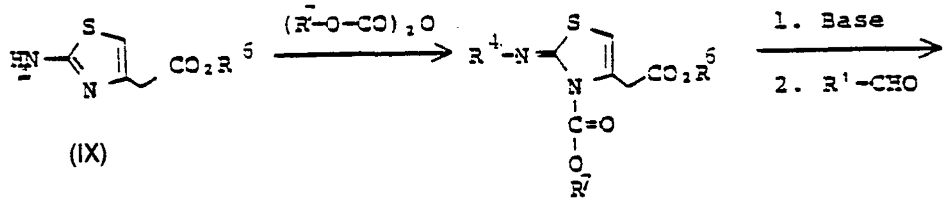


(IX) (X)

(Bei diesem Verfahren zur Herst. von II ist $R^4=R^7—O—CO$)

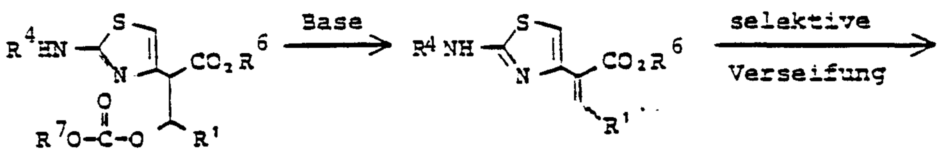


(XI) (XII)

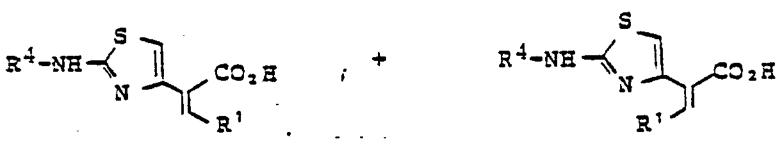

(II) (IIa)

[ (IIa) 1. Silylierung 2. Base (II) + (IIa) Trennung (II) ]

(II)

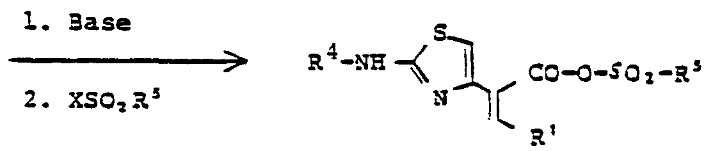


(VII) (III)

Zunächst werden die Verbindungen der Formel IX (siehe z. B. E. Campaigne und T. P. Selby, J. Heterocycl. Chemie 17 (1980), 1255) zu den Verbindungen der Formel X umgesetzt werden.

Es bedeutet

$R^4$ eine Aminschutzgruppe wie z. B. Acetyl, Benzoyl, Formyl, Trichloracetyl, Benzyloxycarbonyl, Methyloxycarbonyl oder tert.-Butyloxycarbonyl ist

$R^6$ und $R^7$ verschieden oder gleich sein können und einen gegebenenfalls substituierten Alkyl-, Cycloalkyl-, Alkenyl-, Cyclolkenyl-, Aryl- oder Heterocyclylrest bedeuten können, wobei Heteroatome als Substituenten der Reste sowie Doppelbindungen in den Alkenyl- und Cycloalkenylresten mindestens durch ein C-Atom von der Oxycarbonylgruppe getrennt sind.

Besonders sind

$R^6$ und $R^7$ ein gegebenenfalls substituierter Alkylrest mit 1-15 C-Atomen, ein gegebenenfalls substituierter Alkenylrest mit 3-15 C-Atomen, ein gegebenenfalls substituierter Cycloalkylrest mit 3-10 C-Atomen, ein gegebenenfalls substituierter Cycloalkenylrest mit 5-10 C-Atomen, ein gegebenenfalls substituierter Arylrest mit 1 bis 3 Ringen oder ein gegebenenfalls substituierter Heterocyclylrest mit 1-3

Ringen, die bis zu 5 Stickstoff-, Schwefel- oder Sauerstoffatome enthalten können.

Die genannten Alkyl-, Alkenyl-, Cycloalkyl- und Cycloalkenylreste können durch Alkylreste mit 1-4 C-Atomen, O-Alkylreste mit 1-4 C-Atomen, Halogen, vorzugsweise Chlor, gegebenenfalls substituierte Phenylreste, C≡N und $C_1$-$C_5$-Trialkylsilyl substituiert sein.

Alle Aryl- und Heterocyclylreste einschließlich der genannten Phenylreste können durch Alkyl-, O-Alkyl-, S-Alkyl-, Alkyloxycarbonyl-, Halogen- und Phenylreste substituiert sein, wobei alle Alkylreste 1 bis 4 C-Atome haben können, sowie durch Nitro und C≡N.

Wenn die Reste $R^6$ und/oder $R^7$ substituiert sind, vorzugsweise durch die obengenannten Substituenten, so können sie 1-5, vorzugsweise 1 oder 2 Substituenten tragen.

Besonders vorteilhaft für das Verfahren ist es, wenn

$R^4$ = eine basenstabile, im Sauren abspaltbare Schutzgruppe wie z. B. tert.-Butoxycarbonyl ist, und wenn

$R^6$ = ein basisch verseifbarer Rest wie z. B. Methyl oder Ethyl ist.

Die Verbindungen der Formel X erhält man, indem man die an sich bekannten Verbindungen der Formel IX in einem geeigneten Lösungsmittel mit einem Pyrokohlensäureester der Formel $R^7$—O—CO—O—CO—O—$R^7$ reagieren läßt.

Als Lösungsmittel eignen sich besonders aprotische, polare Lösungsmittel wie z. B. Acetonitril, Dimethylformamid, Hexamethylphosphorsäuretriamid oder Dimethylsulfoxid, besonders die beiden letzten. Die Reaktion läuft besonders vorteilhaft bei Zimmertemperatur oder bei niedrigeren Temperaturen z. B. 0 bis —50 °C ab, wobei man die Komponenten 1-7 Tage miteinander reagieren läßt. Der Pyrokohlensäureester wird im allgemeinen mit 2-2,5 Moläquivalenten eingesetzt.

Zur Herstellung der Verbindungen der Formel XI versetzt man die Verbindungen der Formel X in einem geeigneten Lösungsmittel bei tiefen Temperaturen mit 1 bis 1,1 Äquivalenten einer Base und gibt anschließend 1 bis 1,2 Äquivalente eines Aldehyds der Formel $R^1$—CHO hinzu.

Als Lösungsmittel für die Reaktion können z. B. Dimethylformamid, Dimethylsulfoxid, Diethylether, Tetrahydrofuran oder Toluol — bevorzugt Tetrahydrofuran — und als Base Alkoholate, Hydride, Amide oder Metallorganyle — bevorzugt Kalium-tert.-Butylat, Lithiumdiisopropylamid und Butyllithium — verwendet werden. Zur Durchführung der Reaktion gibt man die Base bei —50 bis —80 °C zu einer Lösung von X und fügt anschließend bei —50 bis —60 °C den Aldehyd hinzu und rührt ca. 12 Stunden bei —50 bis —60 °C. Zur Isolierung der Produkte der Formel XI wird der Ansatz neutralisiert und aufgearbeitet.

In den Verbindungen der Formel XI haben $R^4$, $R^6$ und $R^7$ die bei den Verbindungen der Formel X aufgeführten Bedeutungen und $R^1$ hat die eingangs genannte Bedeutung.

Zur Durchführung des Verfahrens zur Herstellung der Verbindungen der Formel I ist es nicht notwendig, die Verbindungen der Formel XI zu isolieren. Es ist vielmehr zweckmäßig, sie in situ direkt in die Verbindungen der Formel XII umzuwandeln. Hierzu reicht es im allgemeinen aus, den Ansatz nach Zugabe des Aldehyds $R^1$—CHO auf Raumtemperatur erwärmen zu lassen und über Nacht bei Raumtemperatur zu rühren. Sollte bis dahin die Eliminierung von XI nach XII nicht vollständig sein, gibt man 1 bis 1,2 Äquivalente einer Base — wie z. B. ein Hydrid, ein Alkoholat oder ein Amid — besonders Kalium-tert.-butylat hinzu und rührt etwa 10 h bei Raumtemperatur.

Hatte man dagegen die Verbindung der Formel XI zuvor isoliert, so gibt man zur Herstellung der Verbindungen der Formel XII zu einer Lösung der Verbindungen der Formel XI in einem geeigneten Lösungsmittel 1,1 bis 2,2 Äquivalente einer Base. Als Lösungsmittel und als Base können die bei der Umsetzung von X nach XI genannten Verwendung finden, bevorzugt Tetrahydrofuran und Kalium-tert.-butylat.

Man erhält die Verbindungen der Formel XII als E/Z-Isomerengemische, die sich z. B. durch Umkristallisieren oder durch Säulenchromatographie an Kieselgel trennen lassen.

In den Verbindungen der Formel XII haben $R^1$, $R^4$ und $R^6$ die gleiche Bedeutung wie in den Verbindungen der Formel XI.

Zur Herstellung der Z-Carbonsäuren der Formel II kann man die Z-Ester, die man durch Trennung des E/Z-Isomerengemische der Ester der Formel XII erhalten kann, verseifen. Es ist aber für die Durchführung des Verfahrens zur Herstellung der Verbindungen der Formel I günstiger, das E/Z-Isomerengemisch der Ester der Formel XII in der Weise selektiv zu verseifen, daß man unter milden Bedingungen erst die E-Ester in die E-Carbonsäuren der Formel II a überführt und abtrennt und anschließend die verbleibenden Z-Ester, in denen die Estergruppe sterisch stärker abgeschirmt ist, unter drastischen Bedingungen zu den Z-Carbonsäuren der Formel II verseift.

Die milden Verseifungsbedingungen, die zu den E-Carbonsäuren II a führen, sind z. B. Ethanol/2 N Natronlauge/Raumtemperatur/24 h. Zweckmäßigerweise führt man die Verseifung in der Art durch, daß man nach der Überführung der Verbindungen der Formel XI in die Verbindungen der Formel XII direkt zu dem Reaktionsansatz 2 N Natronlauge gibt, und bei Raumtemperatur oder unter leichtem Erwärmen so lange rührt, bis die E-Ester verseift sind. Dann trennt man durch Extraktion im Alkalischen die Z-Ester von dem Ansatz ab und verseift sie unter drastischeren Bedingungen.

Drastischere Verseifungsbedingungen sind z. B. Ethanol/2 N Natronlauge/24 h Rückfluß — eventuell auch stärkere Natronlauge oder höherer siedende Lösungsmittel wie z. B. Dioxan.

Auf diese Weise erhält man die gewünschten Z-Carbonsäuren der Formel II und die E-Carbonsäuren

der Formel II a. Letztere lassen sich nach Überführung in die Silylester — z. B. mit Bistrimethylsilylacetamid — in einem geeigneten Lösungsmittel — z. B. Diethylether oder Tetrahydrofuran — mit einer Base wie Kalium-tert.-butylat und anschließender Hydrolyse mit verdünnter Säure wieder in ein Gemisch der E-Carbonsäuren der Formel II a und der Z-Carbonsäuren der Formel II überführen.

Aus diesem E/Z-Isomerengemisch lassen sich die Z-Carbonsäuren der Formel II z. B. durch Kristallisation oder durch Trennung an einem Ionenaustauscher rein isolieren.

Die Trennung mit Hilfe von Ionenaustauschern ist einfach, da die Z-Carbonsäuren der Formel II sehr viel stärker sauer sind als die E-Carbonsäuren der Formel II a. So werden die E-Carbonsäuren der Formel II a bereits mit Methanol von schwach-basischen Ionenaustauschern eluiert, die Z-Carbonsäuren der Formel II dagegen erst nach Zusatz von Elektrolyten, z. B. 2 N Natronlauge. Unter schwach basischen Ionenaustauschern sind solche Ionenaustauscher in fester oder flüssiger Form zu verstehen, die tertiäre Aminogruppen enthalten wie z. B. Lewatit MP 62.

In den Verbindungen der Formel II und II a haben $R^1$ und $R^4$ die gleiche Bedeutung wie bei den Verbindungen der Formel XII. Zusätzlich kann $R^4$=H sein, wenn vor der Verseifung in den Verbindungen der Formel XII $R^4$ eine alkalisch verseifbare Schutzgruppe wie z. B. Acetyl war. Für die Durchführung des Verfahrens zur Herstellung der Verbindungen der Formel I ist es aber zweckmäßiger, wenn $R^4$ eine Schutzgruppe ist, die unter den Verseifungsbedingungen stabil ist — bevorzugt tert.-Butyloxycarbonyl.

Für die Kupplung von Carbonsäuren an 7-Aminocephalosporansäuren sind in der Cephalosporinchemie eine große Anzahl von Methoden bekannt, die sich letztlich aus der Peptidchemie ableiten. Bei den Versuchen zur Knüpfung der Amidbindung zwischen den Z-Carbonsäuren der Formel II und den Cephalosporansäuren der Formel IV versagen diese Methoden jedoch oder führen nur zu sehr schlechten Ausbeuten, insbesondere dann, wenn $R^1$ ein Alkylrest ist. Die Gründe hierfür sind in der großen sterischen Hinderung der Carboxygruppe in den Carbonsäuren der Formel II durch den Rest $R^1$ sowie in der ausgeprägten Neigung des Restes $R^1$, nach Aktivierung der Carboxylfunktion — z. B. Überführung in das Säurechlorid — in die E-Form zu isomerisieren.

Man kann aber die Z-Carbonsäuren der Formel II auf einfache, schonende und billige Weise aktivieren, wenn man sie bei tiefen Temperaturen in die gemischten Anhydride der Formel III überführt.

Solche gemischten Anhydride der Formel III können hergestellt werden, indem man die Carbonsäure II und ein geeignetes Amin in äquimolaren Mengen in einem geeigneten Lösungsmittel löst und mit 1 bis 1,05 Äquivalenten eines Sulfonsäurederivates der Formel VII reagieren läßt.

Als Lösungsmittel eignen sich alle Solventien, die unter den Reaktionsbedingungen stabil sind, wie z. B. Diethylether, Tetrahydrofuran, Acetonitril, Aceton, Methylenchlorid, Chloroform oder Dimethylformamid.

Als Amin eignen sich tertiäre Amine wie z. B. Triethylamin oder Tributylamin aber auch sterisch gehinderte sekundäre Amine wie z. B. Diisopropylamin.

Die Umsetzungen können bei Temperaturen zwischen —80 °C und Raumtemperatur durchgeführt werden, wobei tiefe Temperaturen eine Isomerisierung der Substituenten an der Doppelbindung vermeiden. Vorteilhaft werden die Reaktionen bei —20 bis —50 °C bei einer Reaktionsdauer von 10 min. bis 10 h durchgeführt.

Die Verbindungen der Formel III können isoliert werden, indem man z. B. in Tetrahydrofuran als Lösungsmittel und mit Triethylamin als Base arbeitet, das gebildete Triethylminhydrochlorid absaugt und das Lösungsmittel im Vakuum abdestilliert. Es ist aber zweckmäßiger, die erhaltenen Lösungen der Verbindungen der Formel III direkt mit den Cephalosporanaten der Formel IV umzusetzen. Hierzu löst man die Cephalosporanaten der Formel IV oder deren Salze in einem geeigneten Lösungsmittel mit 1-4 Äquivalenten eines Amins, kühlt die Lösung auf die gewünschte, anschließende Reaktionstemperatur vor und gibt bei dieser Temperatur diese Lösung zu der oben beschriebenen Lösung der Verbindung der Formel III. Zur Vermeidung einer Isomerisierung des Restes $R^1$ in den Reaktionsprodukten der Formel V führt man die Reaktion zweckmäßigerweise bei —60 bis —30 °C durch und läßt den Ansatz über Nacht auf Raumtemperatur kommen.

Zum Lösen der Verbindungen der Formel IV können die bei der Herstellung der Verbindungen der Formel III genannten Lösungsmittel sowie als Base die dort genannten Amine verwendet werden.

Im allgemeinen ist die Löslichkeit der Verbindungen der Formel IV in diesen Lösemitteln sehr beschränkt, so daß man hier vorteilhaft in ansich bekannter Weise silyliert oder mit Wasser als Lösemittel arbeitet.

Es ist besonders vorteilhaft, die Carbonsäuren VI ohne Schutzgruppe mit den Sulfonsäurederivaten VII in die gemischten Anhydride der Formel VIII zu überführen und diese mit IV direkt zu den Verbindungen der Formel I umzusetzen.

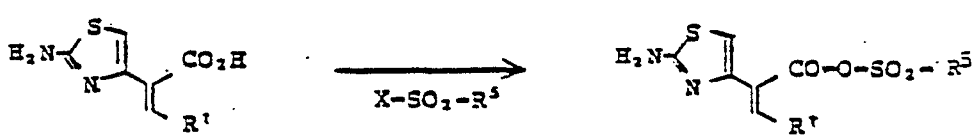


(VI) (VII) (VIII)

$$(VIII) + (IV) \xrightarrow{Base} (I)$$

Es bedeuten

X = Cl, Br, $OSO_2R^5$

$R^5$ = einen Alkylrest mit 1-10 C-Atomen, der gegebenenfalls durch Fluor, chlor, CN, Phenyl, Alkyloxycarbonyl, Alkyloxy oder Alkyl substituiert sein kann, wobei letztere Alkylreste 1-4 C-Atome tragen können, oder ein Phenylrest, der gegebenenfalls durch Fluor, Chlor, Brom, CN, Alkyl, Alkyloxy, Alkylthio, Alkyloxycarbonyl — wobei letztere Alkylgruppen 1-4 C-Atome tragen können — Nitro, Trifluormethyl und Phenyl substituiert sein kann.

Wenn $R^5$ substituiert ist, sind vorzugsweise 1-3 Substituenten, vorzugsweise die genannten vorhanden.

Ganz besonders bevorzugt stellt $R^5$ einen Methyl- oder p-Tolylrest dar.

Die gemischten Anhydride der Formel VIII werden in Analogie zu den Anhydriden der Formel III hergestellt, indem man die Carbonsäuren der Formel VI und 1-1,4 Äquivalente eines Amins in einem Lösungsmittel löst und mit 1 bis 1,2 Äquivalenten eines Sulfonsäurederivates der Formel VII reagieren läßt.

Als Lösungsmittel eignen sich alle Solventien, die unter den Reaktionsbedingungen stabil sind, wie z. B. Diethylester, Tetrahydrofuran, Acetonitril, Aceton, Methylenchlorid, Chloroform oder Dimethylformamid.

Als Amin eignen sich tertiäre Amine wie z. B. Triethylamin oder Tributylamin aber auch sterisch gehinderte sekundäre Amine wie z. B. Diisopropylamin.

Die Umsetzungen können bei Temperaturen zwischen —80 °C und Raumtemperatur durchgeführt werden, wobei tiefe Temperaturen eine Isomerisierung der Substituenten an der Doppelbindung vermeiden. Vorteilhaft wird die Umsetzung mit $Cl-SO_2CH_3$, in Dimethylformamid bei —40 bis —60 °C durchgeführt.

Zum Lösen der Verbindungen der Formel IV können die bei der Herstellung der Verbindungen der Formel VIII genannten Lösungsmittel sowie als Base die dort genannten Amine verwendet werden.

Im allgemeinen ist die Löslichkeit der Verbindungen der Formel IV in diesen Lösungsmitteln sehr beschränkt, sodaß man hier vorteilhaft in an sich bekannter Weise silyliert oder mit Wasser als Lösungsmittel arbeitet.

Die Verbindungen der Formel VI erhält man, indem man von den Verbindungen der Formel II die Schutzgruppe $R^4$ abspaltet — z. B. die Boc-Schutzgruppe mit Trifluoressigsäure.

Ein weiteres Verfahren zur Herstellung der Verbindungen der Formel I ist die Umsetzung von Cephalosporinen der Formel XII mit Pyridinen der Formel XIII, wobei

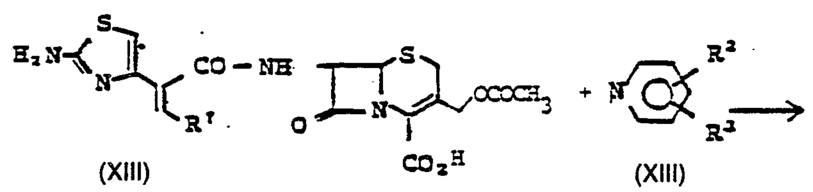 (I)

(XIII) (XIII)

$R^1$-$R^3$ die bei den Verbindungen der Formel I genannte Bedeutung aufweisen. Die Umsetzung wird in einem polaren organischen Lösungsmittel wie z. B. Dimethylformamid oder in Wasser — bevorzugt in Wasser mit einem Überschuß des Pyridins — im allgemeinen 10 Äquivalente bei Temperaturen zwischen 40 und 95 °C und Reaktionszeiten zwischen 5 min. und 6 h durchgeführt. Zur Isolierung der Produkte der Formel I ist es vorteilhaft, nach Extraktion des Pyridins das erhaltene Rohprodukt an einem Harz wie z. B. Diaion HP 20 oder XAD 7 oder auch an Cellulose zu chromatographieren.

Die Verbindungen der Formel XII können erhalten werden in analoger Weise wie die erfindungsgemäßen Verbindungen der Formel I. Hierzu wird lediglich bei der Kupplung der Verbindungen der Formel VIII statt des Cephalosporanats der Formel IV 7-Aminocephalosporansäure eingesetzt.

Die erfindungsgemäßen Verbindungen weisen eine starke und breit antimikrobielle Wirksamkeit besonders gegen gram-negative und gram-positive Bakterien auf. Diese Eigenschaften ermöglichen ihre Verwendung als chemotherapeutische Wirkstoffe in der Medizin. Mit ihrer Hilfe können die durch gramnegative und grampositive Bakterien und bakterienähnliche Mikroorganismen hervorgerufenen Erkrankungen verhindert, gebessert und/oder geheilt werden.

Besonders wirksam sind die erfindungsgemäßen Verbindungen gegen Bakterien und bakterienähnliche Mikroorganismen.

Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden :

Micrococcaceae, wie Staphylokokken, z. B. Staphylococcus aureus, Staph. epidermidis, Staph. aerogenes und Gaffkya tetragena (Staph. = Staphylococcus) ;

Lactobacteriaceae, wie Streptokokken, z. B. Streptococcus pyogenes, α- bzw. β-hämolysierende Streptokokken, nicht (γ-)-hämolysierende Streptokokken, Str. viridans, Str. faecalis (Enterokokken) und Dipolococcus pneumoniae (Pneumokokken) (Str.=Streptococcus) ;

Enterobacteriaceae, wie Escherichiae-Bakterien der Coli-Gruppe : Escherichia-Bakterien, z. B. Escherichia coli, Enterobacter-Bakterien, z. B. E. aerogenes, E. cloacae, Klebsiella-Bakterien, z. B. K. pneumoniae, Serratia, z. B. Serratia marvescens (E. = Enterobacter) (K. = Klebsiella), Proteae-Bakterien der Proteus-Gruppe : Proteus, z. B. Proteus vulgaris, Pr. morganii, Pr. rettgeri, Pr. mirabillis (Pr. = Proteus) ;

Pseudomonadaceae, wie Pseudomonas-Bakterien, z. B. Pseudomonas aeruginosa, (Ps. = Pseudomonas) ;

Bacteroidaceae, wie Bacteroides-Bakterien, z. B. Bacteroides fragilis, (B. = Bacteroides).

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können seien beispielsweise genannt :

Erkrankungen der Atemwege und des Rachenraumes ;

Otitis ; Pharyngitis ; Pneumonie ; Peritonitis ; Pyelonephritis ; Cystitis ; Endocarditis ; Systeminfektionen ; Bronchitis ; Arthritis ; lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichtoxischen, inerter pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, sie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Zum parenteralen Applikation können die Lösungen auch in steriler und blutisotonischer Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitonal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 1 bis etwa 1 000, vorzugsweise 1 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 1 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäßen Verbindungen können zum Zwecke der Erweiterung des Wirkungsspektrums mit einem anderen β-Lactamantibiotikum oder auch mit Aminoglykosidantibiotica, wie z. B. Gentamicin, Sisomicin, Kanamicin, Amikacin oder Tobramicin kombiniert werden.

## Beispiele

1. 7-[1-(2-tert.-Butoxycarbonylaminothiazol-4-yl)-1(Z)-propencarboxamido]-3-pyridiniummethyl-3-cephem-4-carboxylat

5 mMol (1, 42 g) 1-(2-tert.-Butoxycarbonylaminothiazol-4-yl)-1(Z)-propencarbonsäure und 5,5 mMol (0,76 ml) Triethylamin wurden in 30 ml wasserfreiem Dimethylformamid gelöst, auf —55 °C abgekühlt, 5,1 mMol (0,4 ml) Methansulfonsäurechlorid hinzugeben und eine halbe Stunde bei —55 °C gerührt.

Die so hergestellte —55 °C kalte Lösung wurde in einem Guß auf eine Lösung von 4 mMol (1,16 g) 7-Amino-3-pyridiniummethyl-3-cephem-4-carboxylat und 4 mMol (0,55 ml) Triethylamin in 2 ml Wasser gegossen, man ließ auf Raumtemperatur erwärmen und hielt dabei den pH mit Triethylamin auf 8-9.5.

Nach 30 min. wurde der Ansatz i. Vak. einrotiert, der Rückstand in wenig Wasser gelöst, ein pH von 3-4 eingestellt, die Lösung mit Essigester extrahiert und die wäßrige Lösung lyophilisiert.

2. 7-[1-(2-tert.-Butoxycarbonylaminothiazol-4-yl)-1(Z)-butencarboxamido]-3-pyridiniummethyl-3-cephem-4-carboxylat

Die Herstellung erfolgte analog Beispiel 1 aus 1-(2-tert.-Butoxycarbonylaminothiazol-4-yl)-1(Z)-butencarbonsäure.

3. 7-[1-(2-tert.-Butoxycarbonylaminothiazol-4-yl)-1(Z)-pentencarboxamido]-3-pyridiniummethyl-3-cephem-4-carboxylat

Die Herstellung erfolgte analog Beispiel 1 aus 1-(2-tert.-Butoxycarbonylaminothiazol-4-yl)-1(Z)-pentencarbonsäure.

4. 7-[1-(2-tert-Butoxycarbonylaminothiazol-4-yl)-1(Z)-heptencarboxamido]-3-pyridiniummethyl-3-cephem-4-carboxylat

Die Herstellung erfolgte analog Beispiel 1 aus 1-(2-tert.-Butoxycarbonylaminothiazol-4-yl)-1(Z)-heptencarbonsäure.

5. 7-[1-(2-tert.-Butoxycarbonylaminothiazol-4-yl)-1(Z)-propencarboxamido]-3-(4-aminocarbonylpyridiniummethyl)-3-cephem-4-carboxylat

Die Herstellung erfolgte analog Beispiel 1 aus 1-(2-tert.-Butoxycarbonylaminothiazol-4-yl)-1(Z)-propencarbonsäure und 7-Amino-3-(4-aminocarbonylpyridiniummethyl)-3-cephem-4-carboxylat.

6a) 7-[1-(2-Aminothiazol-4-yl)-1(Z)-propencarboxamido]-3-pyridiniummethyl-3-cephem-4-carboxylat

41 mMol (7,5 g) 1-(2-Aminothiazol-4-yl)-1(Z)-propencarbonsäure und 45 mMol (6,3 ml) Triethylamin wurden in 200 ml wasserfreiem Dimethylformamid gelöst, auf —55 °C abgekühlt, 42 mMol (3,3 ml) Methansulfonsäurechlorid hinzugegeben und 30 min. bei —55 °C gerührt.

Danach wurde die —55 °C kalte Lösung in einem Guß auf eine Lösung von 31 mMol (11,2 g) 7-Amino-3-pyridiniummethyl-3-cephem-4-carboxylat, 31 mMol (4,3 ml) Triethylamin in 20 ml Wasser gegeben und unter starkem Rühren auf Raumtemperatur erwärmen gelassen, wobei der pH mit

Triethylamin bei 8-9.5 gehalten wurde.

Zur Aufarbeitung wurde der Ansatz i.Vak. einrotiert, der Rückstand mit Ether verrieben, mehrfach mit Methylenchlorid und Aceton gewaschen.

Um Rest von Salzen und Verunreinigungen zu entfernen, wurde das Produkt durch Chromatographie an Cellulose mit Acetonitril/Wasser 5 : 1 oder durch Absorption an z. B. Diaion HP 20 oder XAD 7 und Desorption mit Wasser/Aceton 90 : 10 gereinigt. Ausbeute ; 10,5 g.

6b) 7-[1-(2-Aminothiazol-4-yl)-1(Z)-propencarboxamido]-3-pyridiniummethyl-3-cephem-4-carboxylat

41 mMol (7,5 g) 1-(2-Aminothiazol-4-yl)-1(Z)-propencarbonsäure und 45 mMol (7,8 ml) Ethyl-diisopropylamin wurden in 100 ml wasserfreiem Dimethylformamid gelöst, auf — 55 °C abgekühlt, 42 mMol (3,3 ml) Methansulfonsäurechlorid hinzugegeben und 40 min. bei — 55 °C gerührt.

Danach wurde die — 55 °C kalte Lösung in einem Guß unter starkem Rühren auf eine Lösung von 31 mMol (11,2 g) 7-Amino-3-pyridinium-methyl-3-cephem-4-carboxylat in 12 ml Wasser gegeben, gleichzeitig 31 mMol (4,3 ml) Triethylamin hinzugegeben und auf Raumtemperatur erwärmen gelassen, wobei der pH mit Triethylamin bei 8.5-9.5 gehalten wurde.

Zur Aufarbeitung wurde der Ansatz nach 10-20 min. in 1,5 l Aceton eingerührt, der Niederschlag abgesaugt und mehrfach mit Methylenchlorid und Aceton gewaschen.

Um Rest von Salzen und Verunreinigungen zu entfernen, wurde das Produkt durch Chromatographie an Cellulose mit Acetonitril/Wasser 5 : 1 oder durch Absorption an z. B. Diaion HP 20 oder XAD 7 und Desorption mit Wasser/Aceton 90 : 10 gereinigt. Ausbeute : 10,5 g.

7. 7-[1-(2-Aminothiazol-4-yl)-1(Z)-propencarboxamido]-3-pyridiniummethyl-3-cephem-4-carboxylat

Das Produkt aus Beispiel 1 wurde bei Raumtemperatur 2 h mit 20 ml Trifluoressigsäure gerührt, i.Vak. einrotiert, der Rückstand mit Ether verrieben, abgesaugt, mit triethylaminhaltigem Methylenchlorid, Methylenchlorid und Aceton gewaschen und wie in Beispiel 6 angegeben gereinigt.

8. 7-[1-(2-Aminothiazol-4-yl)-1(Z)-butencarboxamido]-3-pyridiniummethyl-3-cephem-4-carboxylat

Zur Herstellung wurde das Produkt aus Beispiel 2 analog Beispiel 7 umgesetzt.

9. 7-[1-(2-Aminothiazol-4-yl)-1(Z)-pentencarboxamido]-3-pyridiniummethyl-3-cephem-4-carboxylat

Zur Herstellung wurde das Produkt aus Beispiel 3 analog Beispiel 7 umgesetzt.

10. 7-[1-(2-Aminothiazol-4-yl)-1(Z)-heptencarboxamido]-3-pyridiniummethyl-3-cephem-4-carboxylat

Zur Herstellung wurde das Produkt aus Beispiel 4 analog Beispiel 7 umgesetzt.

11. 7-[1-(2-Aminothiazol-4-yl)-1(Z)-propencarboxamido]-3-(4-aminocarbonylpyridiniummethyl)-3-cephem-4-carboxylat

Zur Herstellung wurde das Produkt aus Beispiel 5 analog Beispiel 7 umgesetzt.

12. 1-(2-Aminothiazol-4-yl)-1(Z)-propencarbonsäure

50 g (0,176 Mol) 1-(2-tert.-Butoxycarbonylaminothiazol-4-yl)-1(Z)-propencarbonsäure und 350 ml Trifluoressigsäure wurden bei 0 °C zusammengegeben und 3 h bei Raumtemperatur gerührt. Die Trifluoressigsäure wurde i.Vak. abgezogen und der Rückstand unter Rühren und Eiskühlung mit gesättigter wäßriger $NaHCO_3$-Lösung bis pH 2 und anschließend mit gesättigter wäßriger $KHCO_3$-Lösung bis pH 3,7-4,0 versetzt. Der Niederschlag wurde abgesaugt, mit Wasser gewaschen und über $P_2O_5$ i.Vak. getrocknet.

Ausbeute 29,5 g (91 %).

13. 7-[1-(2-Aminothiazol-4-yl)-1(Z)-propencarboxamido]-3-(2-methylpyridinium) methyl-3-cephem-4-carboxylat

Die Herstellung erfolgte analog Beispiel 6 aus 3 mMol (552 mg) 1-(2-Aminothiazol-4-yl)-1(Z)-propencarbonsäure und 3 mMol (915 mg) 7-Amino-3-(2-methylpyridinium) methyl-3-cephem-4-carboxylat.

Zur Aufarbeitung wurde der Ansatz auf 450 ml Aceton gegoßen, der entstehende Niederschlag abgesaugt und durch Absorption an Diaion HP 20 und Desorption mit Wasser/Acetonitril 9 : 1 gereinigt. Ausbeute : 377 mg.

$^1H$-NMR ($D_6$-DMSO)

δ = 9.35 (1H, d, J=6 Hz, H-6-Py) ; 9.19 (1H, d, J=8 Hz, NH) ; 8.42 (1H, m, H-4-Py) ; 7.97 (2H, m, H-3,5-Py) ; 6.98 (2H, bs, $NH_2$) ; 6.37 (1H, q, J=8 Hz, C=CH) ; 6.18 (1H, s, Thiazol) ; 5.66 (1H, dd, J=8 Hz, J=5 Hz, H-7-Lactam) ; 5.39 (2H, bs, $CH_2$-Py) ; 5.07 (1H, d, J=5 Hz, H-6-Lactam) ; 3.42 (1H, d, J=18 Hz, S-$CH_2$) ; 3.09 (1H, d, J=18 Hz, S-$CH_2$) ; 2.84 (2H, s, Py-$CH_3$) ; 1.75 (3H, d, J=8 Hz, C=C-$CH_3$).

14. 7-[1-(2-Aminothiazol-4-yl)-1(Z)-propencarboxamido]-3-(3-methylpyridinium) methyl-3-cephem-4-carboxylat

Die Herstellung erfolgte analog Beispiel 13 aus 7-Amino-3-(3-methylpyridinium) methyl-3-cephem-4-carboxylat.
Ausbeute : 370 mg.
$^1$H-NMR ($D_6$-DMSO)
δ = 9.42 (1H, d, J=5 Hz, H-6-Py) ; 9.29 (1H, s, H-2-Py) ; 9.18 (1H, d, J=7 Hz, NH) ; 8.42 (1H, d, J=8 Hz, H-4-Py) ; 8.06 (1H, m, H-5-Py) ; 6.98 (2H, bs, $NH_2$) ; 6.38 (1H, q, J=8 Hz, C=CH) ; 6.17 (1H, s, Thiazol) ; 5.65 (2H, m, H-7-Lactam, $CH_2$-Py) ; 5.09 (1H, d, J=5 Hz, H-6-Lactam) ; 5.06 (1H, d, J=15 Hz, $CH_2$-Py) ; 3.55 (1H, d, J=18 Hz, S-$CH_2$) ; 3.08 (1H, d, J=18 Hz, S-$CH_2$) ; 2.48 (3H, s, Py-$CH_3$) ; 1.75 (3H, d, J=8 Hz ; C=C-$CH_3$).

15. 7-[1-(2-Aminothiazol-4-yl)-1(Z)-propencarboxamido]-3-(4-methylpyridinium) methyl-3-cephem-4-carboxylat

Die Herstellung erfolgte analog Beispiel 13 aus 7-Amino-3-(4-methylpyridinium) methyl-3-cephem-4-carboxylat.
Ausbeute : 360 mg.
$^1$H-NMR ($D_6$-DMSO)
δ = 9.33 (2H, d, J=6 Hz, H-2,6-Py) ; 9.20 (1H, d, J=8 Hz, NH) ; 7.99 (2H, d, J=6 HZ, H-3,5-Py) ; 6.98 (2H, bs, $NH_2$) ; 6.30 (1H, q, J=8 Hz, C=CH) ; 6.18 (1H, s, Thiazol) ; 5.68 (1H, dd, J=8 Hz, J=5 Hz, H-7-Lactam) ; 5.61 (1H, d, J=15 Hz, $CH_2$-Py) ; 5.11 (1H, d, J=5 Hz, H-6-Lactam) ; 5.03 (1H, d, J=15 Hz, $CH_2$-Py) ; 3.55 (1H, d, J=18 Hz, S-$CH_2$) ; 3.04 (1H, d, J=18 Hz, S-$CH_2$) ; 2.60 (3H, s, Py-$CH_3$) ; 1.76 (3H, d, J=8 Hz, C=C-$CH_3$).

16. 7-[1-(2-Aminothiazol-4-yl)-1(Z)-propencarboxamido]-3-(4-äthylpyridinium) methyl-3-cephem-4-carboxylat

Die Herstellung erfolgte analog Beispiel 16 aus 7-Amino-3-(4-äthylpyridinium) methyl-3-cephem-4-carboxylat.
Ausbeute : 140 mg.
$^1$H-NMR ($D_6$-DMSO)
δ = 9.35 (2H, d, J=6 Hz, H-2,6-Py) ; 9.18 (1H, d, J=9 Hz, NH) ; 8.03 (2H, d, J=6 Hz, H-3,5-Py) ; 6.98 (2H, bs, $NH_2$) ; 6.29 (1H, q, J=8 Hz, C=CH) ; 6.18 (1H, s, Thiazol) ; 5.68 (1H, dd, J=9 Hz, J=5 Hz, H-7-Lactam) ; 5.61 (1H, d, J=15 Hz, $CH_2$-Py) ; 5.10 (1H, d, J=5 Hz, H-6-Lactam) ; 5.05 (1H, d, J=15 Hz, $CH_2$-Py) ; 3.54 (1H, d, J=18 Hz, S-$CH_2$) ; 3.04 (1H, d, J=18 Hz, S-$CH_2$) ; 2.89 (2H, q, J=6 Hz, Py-$CH_2$-$CH_3$) ; 1.75 (3H, d, J=8 Hz, C=C-$CH_3$) ; 1.26 (3H, t, J=6 Hz, Py-$CH_2$-$CH_3$).

17. 7-[1-(2-Aminothiazol-4-yl)-1(Z)-propencarboxamido]-3-(2,3-dimethylpyridinium) methyl-3-cephem-4-carboxylat

Die Herstellung erfolgte analog Beispiel 13 aus 7-Amino-3-(2,3-dimethylpyridinium) methyl-3-cephem-4-carboxylat.
Ausbeute : 280 mg.
$^1$H-NMR ($D_6$-DMSO)
δ = 9.19 (1H, d, J=9 Hz, NH) ; 9.16 (1H, d, J=6 Hz, H-6-Py) ; 8.29 (1H, d, J=8 Hz, H-4-Py) ; 7.88 (1H, m, H-5-Py) ; 6.98 (2H, bs, $NH_2$) ; 6.27 (1H, q, J=8 Hz, C=CH) ; 6.17 (1H, s, Thiazol) ; 5.65 (1H, dd, J=9 Hz, J=5 Hz, H-7-Lactam) ; 5.38 (2H, bs, $CH_2$-Py) ; 5.07 (1H, d, J=5 Hz, H-6-Lactam) ; 3.42 (1H, d, J=18 Hz, S-$CH_2$) ; 3.07 (1H, d, J=18 Hz, S-$CH_2$) ; 2.74 (3H, s, Py-2-$CH_3$) ; 2.40 (3H, s, Py-3-$CH_3$) ; 1.75 (3H, d, J=8 Hz, C=C-$CH_3$).

18. 7-[1-(2-Aminothiazol-4-yl)-1-(Z)-propencarboxamido]-3-(2,4-dimethylpyridinium) methyl-3-cephem-4-carboxylat

Die Herstellung erfolgte analog Beispiel 13 aus 7-Amino-3-(2,4-dimethylpyridinium) methyl-3-cephem-4-carboxylat.
Ausbeute : 320 mg.
$^1$H-NMR ($D_6$-DMSO)
δ = 9.20 (1H, d, J=9 Hz, NH) ; 9.17 (1H, d, J=6 Hz, H-6-Py) ; 7.83 (1H, s, H-3-Py) ; 7.81 (1H, d, J=6 Hz, H-5-Py) ; 6.98 (2H, bs, $NH_2$) ; 6.28 (1H, q, J=8 Hz, C=CH) ; 6.17 (1H, s, Thiazol) ; 5.65 (1H, dd, J=9 Hz ; J=5 Hz, H-7-Lactam) ; 5.31 (2H, bs, $CH_2$-Py) ; 5.05 (1H, d, J=5 Hz, H-6-Lactam) ; 3.40 (1H, d, J=18 Hz, S-$CH_2$) ; 3.05 (1H, d, J=18 Hz, S-$CH_2$) ; 2.75 (3H, s, Py-2-$CH_3$) ; 2.50 (3H, s, Py-4-$CH_3$) ; 1.75 (3H, d, J=8 Hz, C=C-$CH_3$).

19. 7-[1-(2-Aminothiazol-4-yl)-1-(Z)-propencarboxamido]-3-(3,4-dimethylpyridinium) methyl-3-cephem-4-carboxylat

Die Herstellung erfolgte analog Beispiel 16 aus 7-Amino-3-(3,4-dimethylpyridinium) methyl-3-cephem-4-carboxylat.

Ausbeute : 200 mg.

$^1$H-NMR ($D_6$-DMSO)

δ = 9.20 (3H, m, NH, H-2,6-Py) ; 7.95 (1H, d, J=6 Hz, H-5-Py) ; 6.98 (2H, bs, $NH_2$) ; 6.29 (1H, q, J=8 Hz, C=CH) ; 6.18 (1H, s, Thiazol) ; 5.66 (1H, dd, J=9 Hz, J=5 Hz, H-7-Lactam) ; 5.58 (1H, d, J=15 Hz, $CH_2$-Py) ; 5.09 (1H, d, J=5 Hz, H-6-Lactam) ; 5.00 (1H, d, J=15 Hz, $CH_2$-Py) ; 3.52 (1H, d, J=18 Hz, S-$CH_2$) ; 3.05 (1H, d, J=18 Hz, S-$CH_2$) ; 2.50 (3H, s, Py-4-$CH_3$) ; 2.36 (3H, s, Py-3-$CH_3$) ; 1.75 (3H, d, J=8 Hz, C=C-$CH_3$).

20. 7-[1-(2-Aminothiazol-4-yl)-1(Z)-propencarboxamido]-3-(3,5-dimethylpyridinium) methyl-3-cephem-4-carboxylat

Die Herstellung erfolgte analog Beispiel 16 aus 7-Amino-3-(3,5-dimethylpyridinium) methyl-3-cephem-4-carboxylat.

Ausbeute : 220 mg.

$^1$H-NMR ($D_6$-DMSO)

δ = 9.20 (2H, s, H-2,6-Py) ; 9.18 (1H, d, J=9 Hz, NH) ; 8.27 (1H, s, H-4-Py) ; 6.97 (2H, bs, $NH_2$) ; 6.28 (1H, q, J=8 Hz, C=CH) ; 6.17 (1H, s, Thiazol) ; 5.65 (1H, dd, J=9 Hz, J=5 Hz, H-7-Lactam) ; 5.60 (1H, d, J=15 Hz, $CH_2$-Py) ; 5.07 (1H, d, J=5 Hz, H-6-Lactam) ; 5.00 (1H, d, J=15 Hz, $CH_2$-Py) ; 3.51 (1H, d, J=18 Hz, S-$CH_2$) ; 3.08 (1H, d, J=18 Hz, S-$CH_2$) ; 2.42 (6H, s, Py-3,5-$CH_3$) ; 1.74 (3H, d, J=8 Hz, C=C-$CH_3$).

21. 7-[1-(2-Aminothiazol-4-yl)-1(Z)-propencarboxamido]-3-(3-chlorpyridinium) methyl-3-cephem-4-carboxylat

Die Herstellung erfolgte analog Beispiel 13 aus 7-amino-3-(3-chlorpyridinium) methyl-3-cephem-4-carboxylat.

Ausbeute : 210 mg.

$^1$H-NMR ($D_6$-DMSO)

δ = 9.92 (1H, s, H-2-Py) ; 9.50 (1H, d, J=6 Hz, H-6-Py) ; 9.22 (1H, d, J=9 Hz, NH( ; 8.88 (1H, d, J=8 Hz, H-4-Py) ; 8.22 (1H, dd, J=8 Hz, J=6 Hz, H-5-Py) ; 6.98 (2H, bs, NH) ; 6.30 (1H, q, J=8 Hz, C=CH) ; 6.18 (1H, s, Thiazol) ; 5.68 (2H, m, H-7-Lactam, $CH_2$-Py) ; 5.11 (1H, d, J=5 Hz, H-6-Lactam) ; 5.08 (1H, J=15 Hz, $CH_2$-Py) ; 3.54 (1H, d, J=18 Hz, S-$CH_2$) ; 3.16 (1H, d, J-18 Hz, S-$CH_2$) ; 1.76 (1H, d, J=8 Hz, C=C-$CH_3$).

22. 7-[1-(2-Aminothiazol-4-yl)-1(Z)-propencarboxamido]-3-(3-aminocarbonylpyridinium) methyl-3-cephem-4-carboxylat

Die Herstellung erfolgte analog Beispiel 13 aus 7-Amino-3-(3-aminocarbonylpyridinium) methyl-3-cephem-4-carboxylat.

Ausbeute : 540 mg.

$^1$H-NMR ($D_6$-DMSO)

δ = 9.78 (2H, m, H-2,6-Py) ; 9.21 (1H, d, J=9 Hz, NH) ; 8.94 (1H, d, J=8 Hz, H-4-Py) ; 8.28 (1H, m, H-5-Py) ; 6.98 (2H, bs, $NH_2$) ; 6.29 (1H, q, J=8 Hz, C=CH) ; 6.18 (1H, s, Thiazol) ; 5.73 (1H, d, J=15 Hz, $CH_2$-Py) ; 5.69 (1H, dd, J=9 Hz, J=5 Hz, H-7-Lactam) ; 5.20 (1H, d, J=15 Hz, $CH_2$-Py) ; 5.11 (1H, d, J= 5 Hz, H-6-Lactam) ; 3.47 (1H, d, J=18 Hz, S-$CH_2$) ; 3.17 (1H, d, J=18 HZ, S-$CH_2$) ; 1.75 (3H, d, J=8 Hz, C=C-$CH_3$).

23. 7-[1-(2-Aminothiazol-4-yl)-1(Z)-propencarboxamido]-3-(4-aminocarbonylpyridinium) methyl-3-cephem-4-carboxylat

Die Herstellung erfolgte analog Beispiel 13 aus 7-Amino-3-(4-aminocarbonylpyridinium) methyl-3-cephem-4-carboxylat.

Ausbeute : 350 mg.

$^1$H-NMR ($D_6$-DMSO)

δ = 9.62 (2H, d, J=6 Hz, H-2,6-Py) ; 9.17 (1H, d, J=9 Hz, NH) ; 8.42 (2H, d, J=6 Hz, H-3,5-Py) ; 6:96 (2H, bs, $NH_2$) ; 6.28 (1H, q, J=8 Hz, C=CH) ; 5.71 (1H, d, J=18 Hz, $CH_2$-Py) ; 5.68 (1H, dd, J=5 Hz ; H-7-Lactam) ; 5.13 (9H, d, J=5 Hz, $CH_2$-Py) ; 5.09 (1H, d, J=5 Hz, H-6-Lactam) ; 3.55 (1H, d, J=18 Hz, S-$CH_2$) ; 3.08 (1H, d, J=18 Hz, S-$CH_2$) ; 1.75 (3H, d, J=8 Hz, C=C-$CH_3$).

24. 7-[1-(2-Aminothiazol-4-yl)-1(Z)-propencarboxamido]-3-pyridiniummethyl-3-cephem-carbonsäure-chlorid-hydrochlorid

Unter Stickstoff wurden 7.0 g (38,6 mM) 1-(2-Aminothiazol-4-yl)-1(Z)-propencarbonsäure in 100 ml DMF gelöst und mit 7.4 ml (42.8 mM) Ethyl-diisopropylamin bei 20 °C versetzt. Man kühlte auf — 55° unter Rühren ab, versetzt mit 3.1 ml (40 mM) Mesylchlorid und rührte 40 Min. nach. 10.7 g (29 mM) 7-Amino-3-pyridiniummethyl-3-cephem-4-carbonsäure-chlorid-hydrat (enthält 0.7 Moläquiv. HCl zusätzlich) wurden inzwischen in 12 ml Wasser unter Zugabe von 5.8 ml (41 mM) Triethylamin auf pH = 7 gestellt und auf 10 °C gekühlt. Die — 55°C kalte DMF-Lösung wurde unter Rühren zugegossen, gleichzeitig hielt man durch Zugabe von Triethylamin den pH-Wert bei 9.5. Nach 10 Min. wurde auf 0 °C gekühlt und mit konz. 12 n HCl auf pH = 1.5-2 gestellt. Nun wurden nochmals 50 ml 12 n HCl zugefügt und anschließend insgesamt 600 ml i-Propanol innerhalb 2 Std. zugegeben, während das Produkt auskristallisierte. Man rührte 1 Std. im Eisbad, saugte scharf ab und wusch mit i-Propanol und Ether. 10.6 g (64 %) Produkt-Dihydrat.

NMR-Signale (in $CD_3OD$) bei

$\delta$ = 9.1 (2H), 8.64 (1H), 8.18 (2H), 6.7 (1H), 6.46 (1H), 5.95 (1H), 5.85 (1H), 5.48 (1H), 5.3 (1H), 3.75 (1H), 3.4 (1H) und 1.98 ppm (3H).

| | | | | | |
|---|---|---|---|---|---|
| ber. | C 42.4 | H 4.4 | Cl 12.5 | N 12.4 | S 11.3 |
| gef. | C 42.0 | H 4.4 | Cl 12.5 | N 12.3 | S 11.2 |

**Patentansprüche** (für die Vertragsstaaten : BE, CH, DE, FR, GB, IT, LI, NL, SE)

1. Verbindungen der Formel I

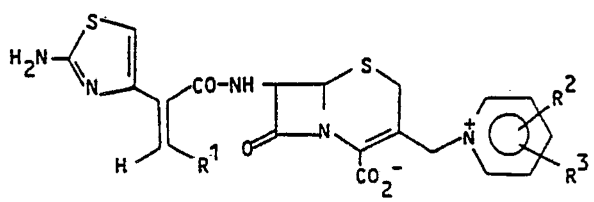

(I)

in der

$R^1$ $C_1$-$C_6$-Alkyl, Phenyl oder Halogen-substituiertes Phenyl, und

$R^2$ und $R^3$ die gleich oder verschieden sein können H, $C_1$-$C_4$-Alkyl, Chlor, Brom, Carbamoyl oder N-$C_1$-$C_4$-Alkylcarbamoyl

bedeuten.

2. Verbindungen nach Anspruch 1, dadurch gekennzeichnet, daß

$R^1$ $C_1$-$C_5$-Alkyl,

$R^3$ Wasserstoff und

$R^2$ H, $C_1$-$C_2$-Alkyl, Carbamoyl, Chlor, Brom

bedeuten.

3. Verbindungen nach Anspruch 1 oder 2, dadurch gekennzeichnet, daß $R_1 = CH_3$ und $R_2 = R_3 = H$ ist.

4. Verfahren zur Herstellung von Verbindungen der Formel I gemäß Anspruch 1, dadurch gekennzeichnet, daß man eine Carbonsäure der Formel VI

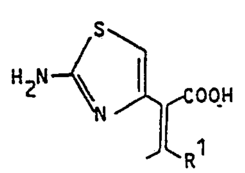

(VI)

in der $R^1$ die in Anspruch 1 angegebene Bedeutung hat, in einem polaren organischen Lösungsmittel bei tiefen Temperaturen mit einer Verbindung der Formel VII

$$X—SO_2—R^5 \qquad (VII)$$

worin

X Chlor, Brom oder $OSO_2$—$R^5$ bedeutet und

$R^5$ einen Alkylrest mit 1-10 C-Atomen, der gegebenenfalls durch Fluor, Chlor, CN, Phenyl, Alkyloxycarbonyl, Alkyloxy oder Alkyl substituiert sein kann, wobei letztere Alkylreste 1-4 C-Atome tragen können, oder einen Phenylrest, der gegebenenfalls durch Fluor, Chlor, Brom, CN, Alkyl, Alkyloxy, Alkylthio, Alkyloxycarbonyl — wobei letztere Alkylgruppen 1-4 C-Atome tragen können — Nitro, Trifluormethyl und Phenyl substituiert sein kann,

darstellt,

zu einem Anhydrid der Formel VIII

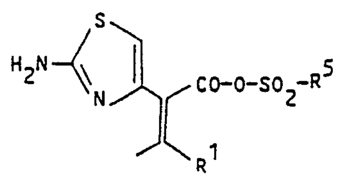

(VIII)

umsetzt und die so erhaltene Lösung zu einer wäßrigen Lösung eines Cephalosporanat der Formel IV

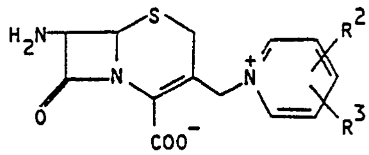

(IV)

oder deren Salz in Gegenwart von 1-4 Äquivalenten einer Base gibt.

5. Verfahren nach Anspruch 4, dadurch gekennzeichnet, daß das polare organische Lösungsmittel Dimethylformamid ist.

6. Verfahren nach den Ansprüchen 4 und 5, dadurch gekennzeichnet, daß die Base Triethylamin ist.

7. Verfahren zur Herstellung von Verbindungen der Formel I gemäß Anspruch 1, dadurch gekennzeichnet, daß man ein Cephalosporin der Formel XII

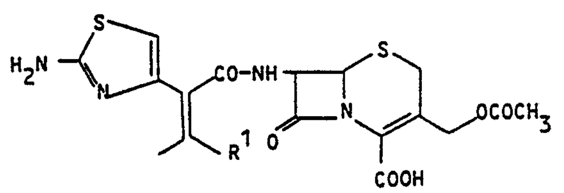

(XII)

worin $R^1$ die in Anspruch 1 angegebene Bedeutung hat in einem organischen oder wäßrigen Lösungsmittel mit einem Pyridin der Formel

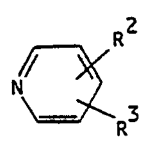

in der $R^2$ und $R^3$ die in Anspruch 1 angegeben Bedeutung haben, bei einer Temperatur von 40 bis 95 °C umsetzt.

8. Pharmazeutische Zusammensetzung enthalten mindestens eine Verbindung gemäß einem der Ansprüche 1-3.

9. Verbindungen der Formel I gemäß einem der Ansprüche 1-3 zur therapeutischen Anwendung.

10. Verwendung von Verbindungen der Formel I gemäß einem der Ansprüche 1-3 zur Herstellung von Arzneimitteln.

**Patentansprüche** (für der Vertragsstaat AT)

1. Verfahren zur Herstellung von Verbindungen der Formel I

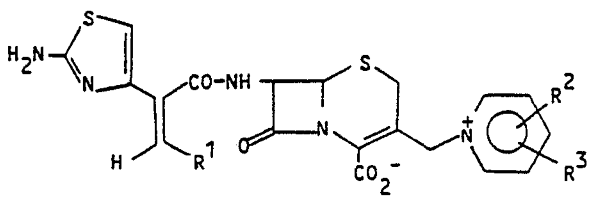

(I)

in der

$R^1$ $C_1$-$C_6$-Alkyl, Phenyl oder Halogen-substituiertes Phenyl, und

$R^2$ und $R^3$ die gleich oder verschieden sein können H, $C_1$-$C_4$-Alkyl, Chlor, Brom, Carbamoyl oder N-$C_1$-$C_4$-Alkylcarbamoyl

bedeuten, dadurch gekennzeichnet, daß man eine Carbonsäure der Formel VI

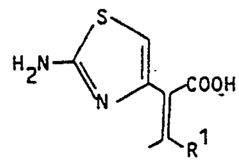

(VI)

in der $R^1$ die in Anspruch 1 angegebene Bedeutung hat, in einem polaren organischen Lösungsmittel bei tiefen Temperaturen mit einer Verbindung der Formel VII

$$X—SO_2—R^5 \qquad (VII)$$

worin

X Chlor, Brom oder $OSO_2—R^5$ bedeutet und

$R^5$ einen Alkylrest mit 1-10 C-Atomen, der gegebenenfalls durch Fluor, Chlor, CN, Phenyl, Alkyloxycarbonyl, Alkyloxy oder Alkyl substituiert sein kann, wobei letztere Alkylreste 1-4 C-Atome tragen können, oder einen Phenylrest, der gegebenenfalls durch Fluor, Chlor, Brom, CN, Alkyl, Alkyloxy, Alkylthio, Alkyloxycarbonyl — wobei letztere Alkylgruppen 1-4 C-Atome tragen können — Nitro, Trifluormethyl und Phenyl substituiert sein kann,

darstellt, zu einem Anhydrid der Formel VIII

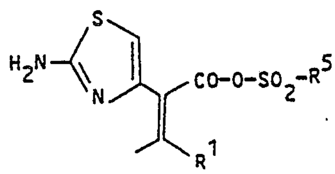

(VIII)

umsetzt und die so erhaltene Lösung zu einer wäßrigen Lösung eines Cephalosporanats der Formel IV

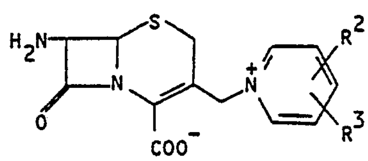

(IV)

oder deren Salz in Gegenwart von 1-4 Äquivalenten einer Base gibt.

2. Verfahren nach Anspruch 1, dadurch gekennzeichnet, daß das polare organische Lösungsmittel Dimethylformamid ist.

3. Verfahren nach den Ansprüchen 1 und 2, dadurch gekennzeichnet, daß die Base Triethylamin ist.

4. Verfahren zur Herstellung von Verbindungen der Formel I gemäß Anspruch 1, dadurch gekennzeichnet, daß man ein Cephalosporin der Formel XII

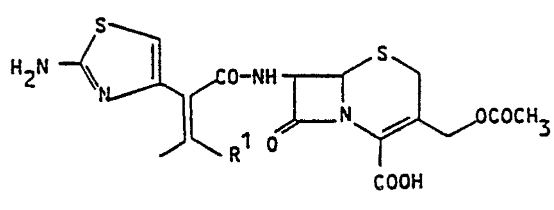

(XII)

worin $R^1$ die in Anspruch 1 angegebene Bedeutung hat, in einem organischen oder wäßrigen Lösungsmittel mit einem Pyridin der Formel

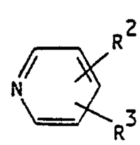

in der $R^2$ und $R^3$ die in Anspruch 1 angegebene Bedeutung haben, bei einer Temperatur von 40 bis 95 °C umsetzt.

**Claims** (for the Contracting States : BE, CH, DE, FR, GB, IT, LI, NL, SE)

1. Compounds of the formula I

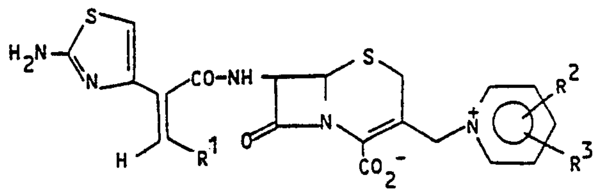

(I)

in which

$R^1$ denotes $C_1$-$C_6$-alkyl, phenyl or halogen-substituted phenyl, and

$R^2$ and $R^3$, which can be identical or different, denote H, $C_1$-$C_4$-alkyl, chlorine, bromine, carbamoyl or N-$C_1$-$C_4$-alkylcarbamoyl.

2. Compounds according to Claim 1, characterised in that

$R^1$ denotes $C_1$-$C_5$-alkyl,

$R^3$ denotes hydrogen and

$R^2$ denotes H, $C_1$-$C_2$-alkyl, carbamoyl, chlorine or bromine.

3. Compounds according to Claim 1 or 2, characterised in that $R_1=CH_3$ and $R_2=R_3=H$.

4. Process for the preparation of compounds of the formula I according to Claim 1, characterised in that a carboxylic acid of the formula VI

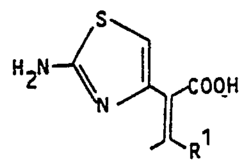

(VI)

in which

$R^1$ has the meaning given in Claim 1, is reacted with a compound of the formula VII

$$X—SO_2—R^5 \quad \text{(VII)}$$

wherein

X denotes chlorine, bromine or $OSO_2—R^5$ and

$R^5$ represents an alkyl radical which has 1-10 carbon atoms and can be optionally substituted by fluorine, chlorine, CN, phenyl, alkyloxycarbonyl, alkyloxy or alkyl, it being possible for the latter alkyl radicals to carry 1-4 C atoms, or represents a phenyl radical which can be optionally substituted by fluorine, chlorine, bromine, CN, alkyl, alkyloxy, alkylthio, alkyloxycarbonyl — it being possible for the latter alkyl groups to carry 1-4 carbon atoms — nitro, trifluoromethyl and phenyl,

in a polar organic solvent at low temperatures to give an anhydride of the formula VIII

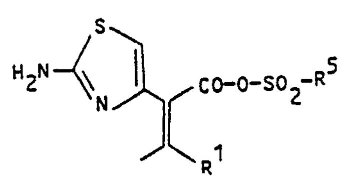

(VIII)

and the solution thus obtained is added to an aqueous solution of a cephalosporanate of the formula IV

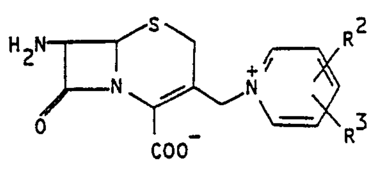

(IV)

or its salt in the presence of 1-4 equivalents of a base.

5. Process according to Claim 4, characterised in that the polar organic solvent is dimethylformamide.

6. Process according to Claims 4 and 5, characterised in that the base is triethylamine.

7. Process for the preparation of compounds of the formula I according to Claim 1, characterised in that a cephalosporin of the formula XII

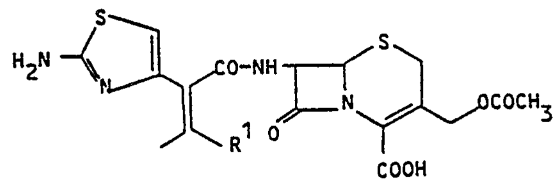

(XII)

wherein $R^1$ has the meaning given in Claim 1, is reacted in an organic or aqueous solvant with a pyridine of the formula

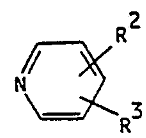

in which $R^2$ and $R^3$ have the meaning given in Claim 1, at a temperature of 40 to 95 °C.

8. Pharmaceutical composition containing at least one compound according to one of Claims 1-3.

9. Compounds of the formula I according to one of Claims 1-3 for therapeutical use.

10. Use of compounds of the formula I according to one of Claims 1-3 for the preparation of medicaments.

**Claims** (for the Contracting State AT)

1. Process for the preparation of compounds of the formula I

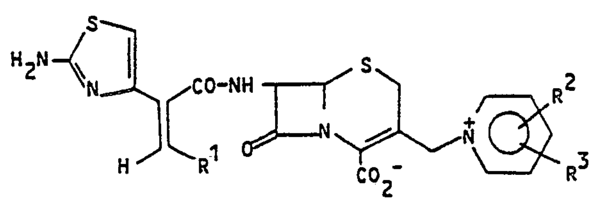

(I)

in which

$R^1$ denotes $C_1$-$C_6$-alkyl, phenyl or halogen-substituted phenyl and

$R^2$ and $R^3$, which can be identical or different, denote H, $C_1$-$C_4$-alkyl, chlorine, bromine, carbamoyl or N-$C_1$-$C_4$-alkylcarbamoyl,

characterised in that a carboxylic acid of the formula VI

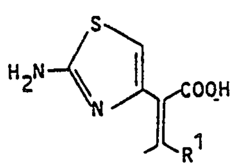

(VI)

in which $R^1$ has the meaning given in Claim 1, is reacted with a compound of the formula VII

$$X—SO_2—R^5 \quad (VII)$$

wherein

X denotes chlorine, bromine or $OSO_2—R^5$ and

$R^5$ represents an alkyl radical which has 1-10 carbon atoms and can be optionally substituted by fluorine, chlorine, CN, phenyl, alkyloxycarbonyl, alkyloxy or alkyl, it being possible for the latter alkyl radicals to carry 1-4 C atoms, or represents a phenyl radical which can be optionally substituted by

fluorine, chlorine, bromine, CN, alkyl, alkyloxy, alkylthio, alkyloxycarbonyl — it being possible for the latter alkyl groups to carry 1-4 carbon atoms — nitro, trifluoromethyl and phenyl, in a polar organic solvent at low temperatures to give an anhydride of the formula VIII

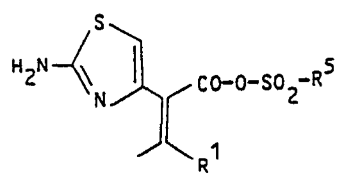

(VIII)

and the solution thus obtained is added to an aqueous solution of a cephalosporanate of the formula IV

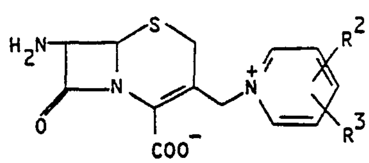

(IV)

or its salt in the presence of 1-4 equivalents of a base.

2. Process according to Claim 1, characterised in that the polar organic solvent is dimethylformamide.

3. Process according to Claims 1 and 2, characterised in that the base is triethylamine.

4. Process for the preparation of compounds of the formula I according to Claim 1, characterised in that a cephalosporin of the formula XII

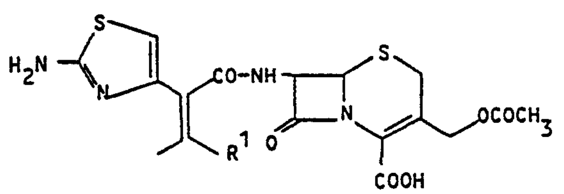

(XII)

wherein $R^1$ has the meaning given in Claim 1, is reacted in an organic or aqueous solvent with a pyridine of the formula

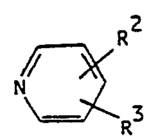

in which $R^2$ and $R^3$ have the meaning given in Claim 1, at a temperature of 40 to 95 °C.

**Revendications** (pour les Etats contractants : BE, CH, DE, FR, GB, IT, LI, NL, SE)

1. Composés de formule I

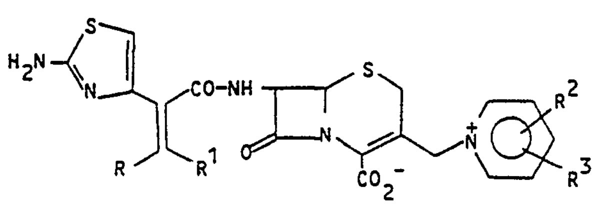

(I)

dans laquelle

$R^1$ représente un groupe alkyle en $C_1$-$C_6$, phényle ou phényle substitué par des halogènes, et

$R^2$ et $R^3$, ayant des significations identiques ou différentes, représentent chacun H, un groupe alkyle en $C_1$-$C_4$, le chlore, le brome, un groupe carbamoyle ou N-(alkyle en $C_1$-$C_4$)-carbamoyle.

2. Composés selon la revendication 1, caractérisés en ce que :
$R^1$ représente un groupe alkyle en $C_1$-$C_5$,
$R^3$ représente l'hydrogène, et
$R^2$ représente H, un groupe alkyle en $C_1$-$C_2$, carbamoyle, le chlore, le brome.

3. Composés selon la revendication 1 ou 2, caractérisés en ce que $R^1 = CH_3$ et $R^2 = R^3 = H$.

4. Procédé de préparation des composés de formule I selon la revendication 1, caractérisés en ce que l'on fait réagir un acide carboxylique de formule VI

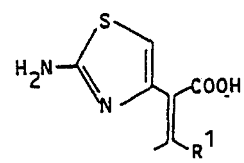

(VI)

dans laquelle $R^1$ a les significations indiquées dans la revendication 1, dans un solvant organique polaire, à basse température, avec un composé de formule VII

$$X—SO_2—R^5 \quad \text{(VII)}$$

dans laquelle
X représente le chlore, le brome ou le groupe $OSO_2—R^5$, et
$R^5$ représente un groupe alkyle en $C_1$-$C_{10}$ éventuellement substitué par le fluor, le chlore, des groupes CN, phényle, alkyloxycarbonyle, alkyloxy ou alkyle, ces derniers groupes alkyle pouvant contenir de 1 à 4 atomes de carbone, ou un groupe phényle éventuellement substitué par le chlore, le fluor, le brome, des groupes CN, alkyle, alkyloxy, alkylthio, alkyloxycarbonyle — ces derniers groupes alkyle pouvant contenir 1 à 4 atomes de carbone — nitro, trifluorométhyle et phényle, ce qui donne un anhydride de formule VIII

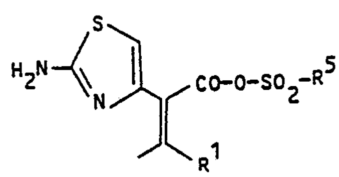

(VIII)

dont on ajoute la solution telle qu'obtenue à une solution aqueuse d'un céphalosporanate de formule IV

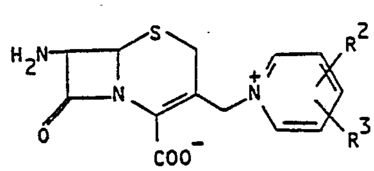

(IV)

ou d'un sel de ce composé, en présence de 1 à 4 équivalents d'une base.

5. Procédé selon la revendication 4, caractérisé en ce que le solvant organique polaire est le diméthylformamide.

6. Procédé selon les revendications 4 et 5, caractérisé en ce que la base est la triéthylamine.

7. Procédé de préparation des composés de formule I selon la revendication 1, caractérisé en ce que l'on fait réagir une céphalosporine de formule XII

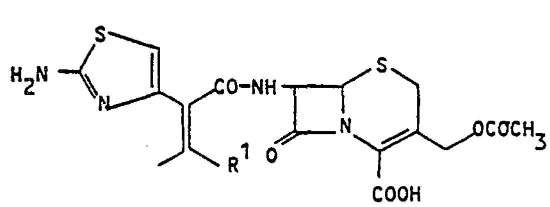

(XII)

dans laquelle $R^1$ a les significations indiquées dans la revendication 1, dans un solvant organique ou aqueux, avec une pyridine de formule

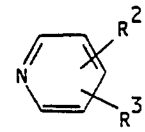

dans laquelle $R^2$ et $R^3$ ont les significations indiquées dans la revendication 1, à une température de 40 à 95 °C.

8. Composition pharmaceutique contenant au moins un composé selon l'une des revendications 1 à 3.

9. Composés de formule I selon l'une des revendications 1 à 3, pour l'utilisation thérapeutique.

10. Utilisation des composés de formule 1 selon l'une des revendications 1 à 3, pour la préparation de médicaments.

**Revendications** (pour l'Etat contractant AT)

1. Procédé de préparation des composés de formule I

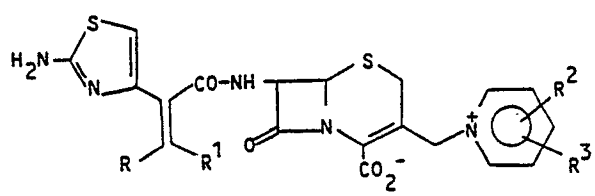

(I)

dans laquelle

$R^1$ représente un groupe alkyle en $C_1$-$C_6$, phényle ou phényle substitué par des halogènes, et

$R^2$ et $R^3$, ayant des significations identiques ou différentes, représentent chacun H, un groupe alkyle en $C_1$-$C_4$, le chlore, le brome, un groupe carbamoyle ou N-(alkyle en $C_1$-$C_4$)-carbamoyle, caractérisé en ce que l'on fait réagir un acide carboxylique de formule VI

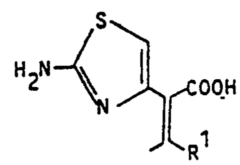

(VI)

dans laquelle $R^1$ a les significations indiquées dans la revendication 1, dans un solvant organique polaire, à basse température, avec un composé de formule VII

$$X—SO_2—R^5 \quad (VII)$$

dans laquelle

X représente le chlore, le brome ou le groupe $OSO_2—R^5$, et

$R^5$ représente un groupe alkyle en $C_1$-$C_{10}$ éventuellement substitué par le fluor, le chlore, des groupes CN, phényle, alkyloxycarbonyle, alkyloxy ou alkyle, ces derniers groupes alkyle pouvant contenir 1 à 4 atomes de carbone, ou un groupe phényle éventuellement substitué par le fluor, le chlore, le brome, des groupes CN, alkyle, alkyloxy, alkylthio, alkyloxycarbonyle — ces derniers groupes alkyle pouvant contenir 1 à 4 atomes de carbone — nitro, trifluorométhyle et phényle, ce qui donne un anhydride de formule VIII

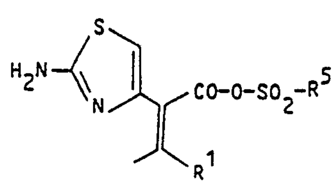

(VIII)

dont on ajoute la solution telle qu'obtenue à une solution aqueuse d'un céphalosporanate de formule IV

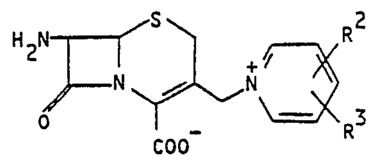

(IV)

ou d'un sel de ce composé en présence de 1 à 4 équivalents d'une base.

2. Procédé selon la revendication 1, caractérisé en ce que le solvant organique polaire est le diméthylformamide.

3. Procédé selon les revendications 1 et 2, caractérisé en ce que la base est la triéthylamine.

4. Procédé de préparation des composés de formule I selon la revendication 1, caractérisé en ce que l'on fait réagir une céphalosporine de formule XII

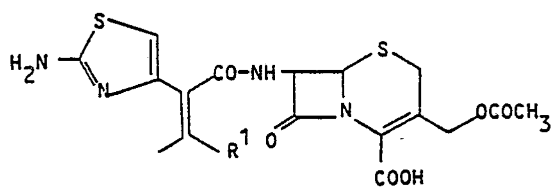

(XII)

dans laquelle $R^1$ a les significations indiquées dans la revendication 1, dans un solvant organique ou aqueux, avec une pyridine de formule

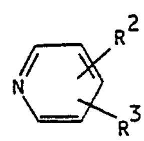

dans laquelle $R^2$ et $R^3$ ont les significations indiquées dans la revendication 1, à une température de 40 à 95 °C.